(19) Europäisches Patentamt
European Patent Office
Office européen des brevets

(11) Veröffentlichungsnummer: **0 217 218**
**A2**

(12) # EUROPÄISCHE PATENTANMELDUNG

(21) Anmeldenummer: 86112757.9

(22) Anmeldetag: 16.09.86

(51) Int. Cl.⁴: C 07 D 487/04
A 01 N 43/10
//(C07D487/04, 249:00, 239:00)

(30) Priorität: 28.09.85 DE 3534651

(43) Veröffentlichungstag der Anmeldung: 08.04.87 Patentblatt 87/15

(84) Benannte Vertragsstaaten: AT BE CH DE FR GB IT LI NL

(71) Anmelder: BAYER AG
Konzernverwaltung RP Patentabteilung
D-5090 Leverkusen 1 Bayerwerk(DE)

(72) Erfinder: Jelich, Klaus, Dr.
Pahlkestrasse 5
D-5600 Wuppertal 1(DE)

(72) Erfinder: Santel, Hans-Joachim, Dr.
Grünstrasse 9a
D-5090 Leverkusen 1(DE)

(72) Erfinder: Schmidt, Robert R., Dr.
Im Waldwinkel 110
D-5060 Bergisch Gladbach 2(DE)

(54) **Triazolo-pyrimidine.**

(57) Die Erfindung betrifft neue Triazolo-pyrimidine der Formel (I).

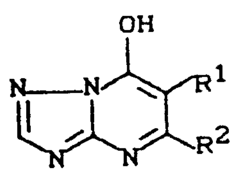

(I)

in welcher
$R^1$ für Wasserstoff oder Alkyl steht und
$R^2$ für Alkoxyalkyl oder Alkylthioalkyl steht,
ein Verfahren zu ihrer Herstellung und ihre Verwendung als Herbizide.

EP 0 217 218 A2

Croydon Printing Company Ltd.

0217218

BAYER AKTIENGESELLSCHAFT
Konzernverwaltung RP
Patentabteilung

5090 Leverkusen, Bayerwerk
KM/ABc
Ia

## Triazolo-pyrimidine

Die Erfindung betrifft neue Triazolo-pyrimidine, ein Verfahren zu ihrer Herstellung und ihre Verwendung als Herbizide.

Es ist bereits bekannt, daß bestimmte Triazol-Verbindungen, wie beispielsweise das 3-Amino-1,2,4-(1H)-triazol oder das 5-Methyl-[1,2,4]-triazolo-[1,5a]-pyrimidin-7-ol herbizide Eigenschaften besitzen (vgl. z.B. Französische Patent Anmeldung 1 433 798 sowie K.H. Büchel "Pflanzenschutz- und Schädlingsbekämpfungsmittel", S. 180 ff., Stuttgart 1977).

Deren herbizide Wirksamkeit gegenüber Problemunkräutern ist jedoch ebenso wie ihre Selektivität gegenüber wichtigen Kulturpflanzen nicht immer in allen Anwendungsbereichen völlig zufriedenstellend.

Es wurden neue Triazolo-pyrimidine der allgemeinen Formel (I),

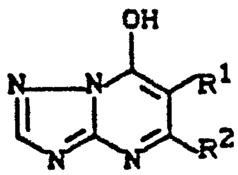

(I)

in welcher

$R^1$ für Wasserstoff oder Alkyl steht und

$R^2$ für Alkoxyalkyl oder Alkylthioalkyl steht,

gefunden.

Weiterhin wurde gefunden, daß man die neuen Triazolopyrimidine der allgemeinen Formel (I),

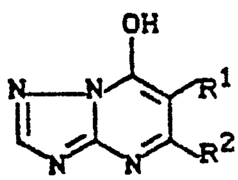

(I)

in welcher

$R^1$ für Wasserstoff oder Alkyl steht und

$R^2$ für Alkoxyalkyl oder Alkylthioalkyl steht,

erhält, wenn man Aminotriazole der Formel (II),

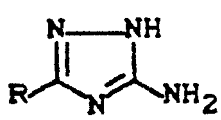

(II)

in welcher

R für Wasserstoff oder für eine Carboxylgruppe steht,

mit β-Ketoestern der Formel (III)

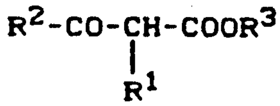

(III)

in welcher

$R^1$ und $R^2$ die oben angegebene Bedeutung haben und

$R^3$ für Alkyl, insbesondere für Methyl oder Ethyl steht,

gegebenenfalls in Gegenwart eines Verdünnungsmittels umsetzt.

Schließlich wurde gefunden, daß die neuen Triazolo-pyrimidine der allgemeinen Formel (I) herbizide, insbesondere auch selektiv-herbizide Eigenschaften besitzen.

Dabei zeigt es sich überraschenderweise, daß die erfindungsgemäßen Triazolo-pyrimidine der allgemeinen Formel (I) neben einer erheblich besseren Wirksamkeit gegenüber bestimmten Problemunkräutern eine deutlich verbesserte Kulturpflanzenselektivität besitzen als beispielsweise die aus dem Stand der Technik bekannte Verbindung 3-Amino-1,2,4-(1H)-triazol, welches eine chemisch und wirkungsmäßig naheliegende Verbindung ist.

Die erfindungsgemäßen Triazolo-pyrimidine sind durch die Formel (I) allgemein definiert. Bevorzugt sind Verbindungen der Formel (I), bei welchen

$R^1$ für Wasserstoff oder für geradkettiges oder verzweigtes Alkyl mit 1 bis 8 Kohlenstoffatomen steht und

$R^2$ für jeweils geradkettiges oder verzweigtes Alkoxyalkyl oder Alkylthioalkyl mit jeweils 1 bis 4 Kohlenstoffatomen in den einzelnen Alkylteilen steht.

Besonders bevorzugt sind Verbindungen der Formel (I), bei welchen

$R^1$ für Wasserstoff, Methyl, Ethyl, n- oder i-Propyl, n-, i-, s- oder t-Butyl steht und

$R^2$ für Methoxymethyl, Methoxyethyl, Ethoxymethyl, Ethoxyethyl, n-Propoxymethyl, i-Propoxymethyl, n-Propoxyethyl, i-Propoxyethyl, Methylthiomethyl, Methylthioethyl, Ethylthiomethyl, Ethylthioethyl, n-Propylthiomethyl, i-Propylthiomethyl, n-Propylthioethyl oder i-Propylthioethyl.

Im einzelnen seien außer den bei den Herstellungsbeispielen genannten Verbindungen die folgenden Triazolopyrimidine der allgemeinen Formel (I) genannt:

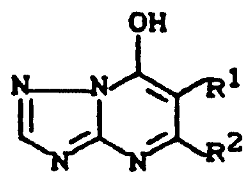

(I)

Tabelle 1

| $R^1$ | $R^2$ | $R^1$ | $R^2$ |
|---|---|---|---|
| $CH_3$ | $-CH_2-CH_2-SCH_3$ | H | $-CH_2-O-CH(CH_3)_2$ |
| $C_2H_5$ | $-CH_2-SCH_3$ | $CH_3$ | $-CH_2O-CH(CH_3)_2$ |
| $CH_3$ | $-CH_2-CH_2-SCH_3$ | H | $-CH_2S-CH(CH_3)_2$ |
| H | $-CH_2-CH_2-SCH_3$ | $CH_3$ | $-CH_2S-CH(CH_3)_2$ |
| H | $-CH_2-CH_2-OCH_3$ | $C_2H_5$ | $-CH_2O-C_2H_5$ |
| H | $-CH_2-CH_2-OC_2H_5$ | $C_2H_5$ | $-CH_2CH_2-OCH_3$ |
| $CH_3$ | $-CH_2-CH_2-OC_2H_5$ | $CH_3$ | $-CH_2-CH_2-OCH_3$ |

Verwendet man beispielsweise 5-Amino-1,2,4-(1H)-triazol-3-ylcarbonsäure und β-Keto-γ-methoxy-buttersäure-ethylester als Ausgangsstoffe, so läßt sich der Reaktionsablauf des Herstellungsverfahrens durch das folgende Formelschema darstellen:

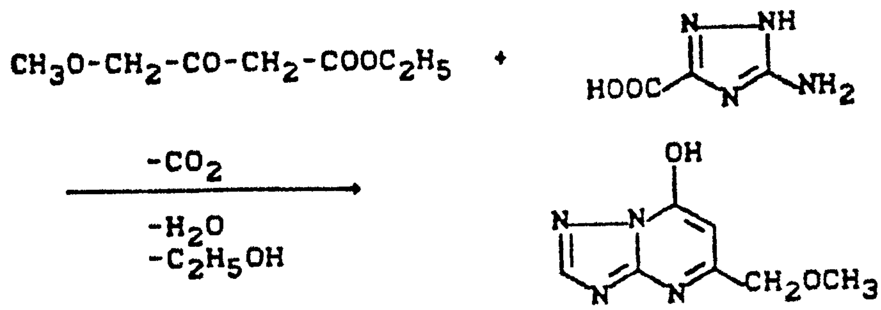


Die zur Durchführung des Herstellungsverfahrens als Ausgangsstoffe benötigten Aminotriazole sind durch die Formel (II) definiert. Die Aminotriazole der Formel (II) sind allgemein bekannte Verbindungen.

Die zur Durchführung des Herstellungsverfahrens weiterhin als Ausgangsstoffe benötigten β-Ketoester sind durch die Formel (III) allgemein definiert. In dieser Formel (III) stehen $R^1$ und $R^2$ vorzugsweise für diejenigen Reste, die im Zusammenhang mit der Beschreibung der erfindungsgemäß verwendbaren Stoffe der Formel (I) bevorzugt für diese Substituenten genannt wurden. $R^3$ steht vorzugsweise für Methyl oder Ethyl. Die β-Ketoester der Formel (III) sind ebenfalls allgemein bekannte Verbindungen der organischen Chemie.

Als Verdünnungsmittel zur Durchführung des Herstellungsverfahrens kommen inerte organische Lösungsmittel in Frage.

Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Alkohole wie Methanol, Ethanol oder Propanol oder Carbonsäuren wie Essigsäure.

Die Reaktionstemperaturen können bei der Durchführung des Herstellungsverfahrens in einem größeren Bereich variiert werden. Im allgemeinen arbeitet man bei Temperaturen zwischen 20 °C und 150°C , vorzugsweise bei Temperaturen zwischen 40 °C und 120 °C.

Zur Durchführung des Herstellungsverfahrens setzt man pro Mol an Aminotriazol der Formel (II) im allgemeinen 1,0 bis 2,5 Mol, vorzugsweise 1,0 bis 1,5 Mol an β-Ketoesterderivat der Formel (III) ein. Die Reaktionsdurchführung, Aufarbeitung und Isolierung der Reaktionsprodukte der Formel (I) erfolgt nach üblichen Verfahren (vgl.z.B. J.Chem.Soc.Perkin Trans.I,1980, 1347-1351).

Die erfindungsgemäß verwendbaren Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäß verwendbaren Stoffe als totale oder selektive Herbizide wirken, hängt im wesentlichen von der angewendeten Menge ab.

Die erfindungsgemäß verwendbaren Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäß verwendbaren Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäß verwendbaren Wirkstoffe mit besonders gutem Erfolg zur selektiven Bekämpfung mono- und dikotyler Unkräuter in mono- und dikotylen Kulturen wie beispielsweise Mais oder Baumwolle einsetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit,

Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäß verwendbaren Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage.

Auch Mischungen mit N,N-Dimethyl-N'-(3-trifluormethylphenyl)-harnstoff; 2-[1-(Ethoxamino)-butyliden]-5-(2-ethylthiopropyl)-1,3-cyclohexadion; 2-{4-[3-Chlor-5-(trifluormethyl)-2-pyridinyl]-oxy-phenoxy}-propansäure bzw. propansäureethylester; 2-[4-(3,5-Dichlorpyrid-2-yloxy)-phenoxy]-propionsäure-2-benzyloxyethylester), -(trimethylsilylmethylester) oder -(2,2-diethoxyethylester); 2-Chlor-4-ethylamino-6-isopropylamino-1,3,5-triazin; 2-Chlor-4-ethylamino-6-(3-cyanopropylamino)-1,3,5-triazin; 2,4-Dichlorphenoxyessigsäure; 2,4-Dichlorphenoxypropionsäure; (2-Methyl-4-chlorphenoxy)-essigsäure; (4-Chlor-2-methylphenoxy)-propionsäure; 6-Chlor- 3-phenyl-pyridazin-4-yl-S-octyl-thiocarbonat; 3,5-Diiod-4-hydroxybenzonitril; 3-Isopropyl-2,1,3-benzothiadiazin-4- on-2,2-dioxid; 2-(3,5-Dichlorphenyl)-2-(2,2,2-trichlorethyl)-oxiran;

2-Methoxy-3,6-dichlor-benzoesäure bzw. -benzoesäure-methylester oder 3,6-Dichlor-2-pyridincarbonsäure sind gegebenenfalls von Vorteil. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäß verwendbaren Wirkstoffe können sowohl vor als auch nach dem Auflaufen der Pflanzen appliziert werden.
Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäß verwendbaren Wirkstoffe geht aus den nachfolgenden Beispielen hervor.

Herstellungsbeispiele:

Beispiel 1:

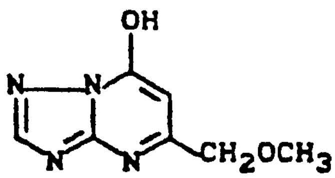

15 g (0,117 Mol) 5-Amino-1,2,4-(1H)-triazol -3-yl-carbonsäure und 34 g (0,233 Mol) β-Keto-γ-methoxy-buttersäureethylester werden in 150 ml Eisessig 12 Stunden lang unter Rückfluß erhitzt. Aus der erkalteten Reaktionsmischung fällt man das Produkt durch Zugabe von 100 ml Diethylether aus und saugt ab.

Man erhält 15 g (71 % der Theorie) an 5-Methoxymethyl-s-triazolo-[1,5-a]-pyrimidin-7-ol vom Schmelzpunkt 257° C.

In entsprechender Weise und gemäß den allgemeinen Angaben zur Herstellung erhält man die folgenden Triazolopyrimidine der allgemeinen Formel (I):

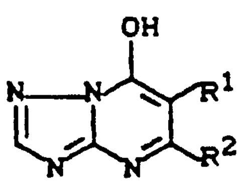

(I)

**Tabelle 2:**

| Bsp. Nr. | $R^1$ | $R^2$ | Schmelz-punkt/$^\circ$C |
|---|---|---|---|
| 2 | $CH_3$ | $-CH_2-OCH_3$ | 227 |
| 3 | H | $-CH_2-SCH_3$ | 215 |
| 4 | $C_2H_5$ | $-CH_2OCH_3$ | 240 |
| 5 | $CH_3$ | $-CH_2-SCH_3$ | |

0217218

Anwendungsbeispiele:

In dem folgenden Anwendungsbeispiel wurde die nachstehend aufgeführte Verbindung als Vergleichsubstanz eingesetzt:

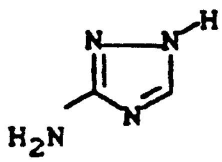

(A)

3-Amino-1,2,4-(1H)-triazol

(bekannt aus K.H. Büchel "Pflanzenschutz-und Schädlingsbekämpfungsmittel", G. Thieme Verlag, Stuttgart 1977, S. 181).

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

Eine deutliche Überlegenheit in der Wirksamkeit, ebenso wie in der Nutzpflanzenselektivität, gegenüber dem Stand der Technik zeigt in diesem Test z.B. die Verbindung gemäß Herstellungsbeispiel 1.

## Patentansprüche

1. Triazolopyrimidine der Formel (I),

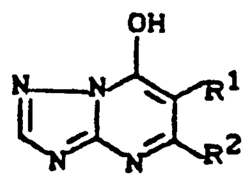

(I)

in welcher

$R^1$ für Wasserstoff oder Alkyl steht und

$R^2$ für Alkoxyalkyl oder Alkylthioalkyl steht.

2. Triazolopyrimidine der Formel (I) gemäß Anspruch 1, dadurch gekennzeichnet, daß

$R^1$ für Wasserstoff oder für geradkettiges oder verzweigtes Alkyl mit 1 bis 8 Kohlenstoffatomen steht und

$R^2$ für jeweils geradkettiges oder verzweigtes Alkoxyalkyl oder Alkylthioalkyl mit jeweils 1 bis 4 Kohlenstoffatomen in den einzelnen Alkylteilen steht.

3. Triazolopyrimidine der Formel (I) gemäß Anspruch 1, dadurch gekennzeichnet, daß

$R^1$ für Wasserstoff, Methyl, Ethyl, n- oder i-Propyl, n-, i-, s- oder t-Butyl steht und

$R^2$ für Methoxymethyl, Methoxyethyl, Ethoxymethyl, Ethoxyethyl, n-Propoxymethyl, i-Propoxymethyl, n-Propoxyethyl, i-Propoxyethyl, Methylthiomethyl, Methylthioethyl, Ethylthiomethyl, Ethylthioethyl, n-Propylthiomethyl, i-Propylthiomethyl, n-Propylthioethyl oder i-Propylthioethyl.

4. Verfahren zur Herstellunbg von Triazolo-pyrimidinen der Formel (I),

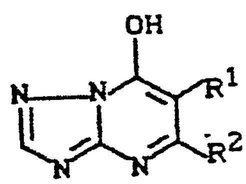

(I)

in welcher

$R^1$ für Wasserstoff oder Alkyl steht und

$R^2$ für Alkoxyalkyl oder Alkylthioalkyl steht,

dadurch gekennzeichnet, daß man Aminotriazole der Formel (II),

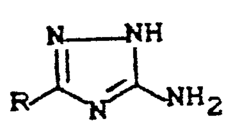

(II)

in welcher

R für Wasserstoff oder für eine Carboxylgruppe steht,

mit β-Ketoestern der Formel (III)

$$R^2\text{-CO-}\underset{R^1}{\underset{|}{CH}}\text{-COOR}^3 \qquad (III)$$

in welcher

$R^1$ und $R^2$ die oben angegebene Bedeutung haben und

$R^3$ für Alkyl, insbesondere für Methyl oder Ethyl steht,

gegebenenfalls in Gegenwart eines Verdünnungsmittels umsetzt.

5. Herbizide Mittel, gekennzeichnet durch einen Gehalt an mindestens einem Triazolo-pyrimidin der Formel (I) gemäß den Ansprüchen 1 bis 4.

6. Verfahren zur Bekämpfung von Unkraut, dadurch gekennzeichnet, daß man Triazolo-pyrimidine der Formel (I) gemäß den Ansprüchen 1 bis 4 auf die Unkräuter und/oder ihren Lebensraum einwirken läßt.

7. Verwendung von Triazolo-pyrimidinen der Formel (I) gemäß den Ansprüchen 1 bis 4 zur Bekämpfung von Unkraut.

8. Verfahren zur Herstellung von herbiziden Mitteln, dadurch gekennzeichnet, daß man Triazolo-pyrimidine der Formel (I) gemäß den Ansprüchen 1 bis 4 mit Streckmittel und/oder oberflächenaktiven Mitteln vermischt.